# EUROPÄISCHE PATENTSCHRIFT

(11) **EP 4 277 576 B1**
(45) Veröffentlichungstag und Bekanntmachung des Hinweises auf die Patenterteilung: **07.08.2024**
(21) Anmeldenummer: 22706034.0
(22) Anmeldetag: 10.02.2022
(51) Int. Cl.: A61F 2/24

(54) **SYSTEM ZUR BEHANDLUNG, PRÄVENTION UND/ODER ZUM ERSATZ EINER HERZKLAPPE, INSBESONDERE EINER ENTZÜNDETEN, INFIZIERTEN, THROMBOSIERTEN ODER DEGENERIERTEN HERZKLAPPE**
SYSTEM FOR TREATING, PREVENTING AND/OR REPLACING A HEART VALVE, IN PARTICULAR AN INFLAMED, INFECTED, THROMBOSED OR DEGENERATED HEART VALVE
SYSTÈME DE TRAITEMENT, DE PRÉVENTION ET/OU DE REMPLACEMENT D'UNE VALVULE CARDIAQUE, EN PARTICULIER D'UNE VALVULE CARDIAQUE ENFLAMMÉE, INFECTÉE, THROMBOSÉE OU DÉGÉNÉRÉE

(30) Priorität: 10.02.2021 DE 102021000811
(43) Veröffentlichungstag der Anmeldung: 22.11.2023
(60) Teilanmeldung: 24156520.9
(73) Patentinhaber: Devie Medical GmbH, 07743 Jena (DE)
(72) Erfinder: FIGULLA, Hans-Reiner, 07749 Jena (DE); SEIDEL, Raphael Andreas, 99428 Weimar (DE); CHAVALLA, Sharath Chandra, 07747 Jena (DE)
(74) Vertreter: Meissner Bolte Partnerschaft mbB
(86) Internationale Anmeldenummer: PCT/EP2022/053299
(87) Internationale Veröffentlichungsnummer: WO 2022/171764

(56) Entgegenhaltungen:
- WO-A1-2020/123267
- WO-A1-2020/234199
- US-A1- 2011 208 298
- US-A1- 2014 371 845
- US-A1- 2017 095 328
- US-A1- 2017 128 199
- US-A1- 2018 125 648
- US-A1- 2020 397 568

## Beschreibung

Gegenwärtig sind zahlreiche Stent-Systeme zum Stützen und Offenhalten von Gefäßen, sowie zur Einbringung bzw. zum Ersatz von Gefäßklappen, wie beispielsweise Herzklappen, vorhanden. Diese Systeme können einerseits chirurgisch, beispielsweise durch eine Operation am offenen Herzen oder minimalinvasiv, und andererseits katheterbasiert direkt über einen arteriellen oder venösen Zugang ins Herzkreislaufsystem eingebracht werden. Die kathetergeführten Implantate besitzen häufig ein ballonexpandierbares Metallgerüst oder einen aus einer Formgedächtnislegierung bestehenden, selbstexpandierbaren Metallrahmen für die Stents bzw. Herzklappen. Weiterhin sind z.T. dünne Schichten oder gewebeartige Strukturen an- oder aufgebracht, die einerseits das Einwachsen des Implantats erleichtern sollen und andererseits das Lumen des Implantats offenhalten und insbesondere bei Herzklappen einen Blutfluss um die Klappe herum (paravalvuläres Leck) verhindern sollen. Die über einen Katheter eingebrachten Herzklappenimplantate zeichnen sich dadurch aus, dass die Belastung für einen Patienten gegenüber dem operativen, den Thorax eröffnenden Eingriff, reduziert ist. Bei Infektionen an einer nativen oder prothetischen Herzklappe, der sogenannten nativen oder prothetischen Endokarditis (NVE, PVE), die mit einer Destruktion der Klappen einhergehen ist ein kathetergeführter Herzklappenersatz absolut kontraindiziert, da dadurch infizierte Bereiche von der Blutzirkulation abgetrennt werden und davon ausgegangen werden kann, dass sich die Infektion ausweitet und sich die Situation für den Patienten verschlechtert. Darüber hinaus kann es beim Einsetzen von herkömmlichen kathetergeführten Herzklappen dazu kommen, dass infektiöse Bakterienauflagerungen an den nativen Herzklappensegeln (sogenannte Vegetationen) in den Blutstrom abreißen und embolisieren.

Der offene chirurgische Eingriff unter Einsatz einer Herz-Lungen-Maschine, der trotz Multimorbidität bei der Mehrzahl der Patienten mit Endokarditis durchgeführt werden muss, kann das infizierte Material, im Gegensatz zum kathetergeführten Herzklappenersatz, entfernen. Allerdings sind diese Operationen bei der infektiösen Endokarditis aufgrund der mit der Endokarditis verbundenen Komorbidität der Patienten mit hohen Eingriffsrisiken verbunden, so dass bei der NVE und PVE eine mittlere Krankenhaussterblichkeit von ungefähr 20% auftritt. Der aktuelle Stand der Technik ist weiterhin in der veröffentlichten Patentanmeldung WO 2020/074130 A1 wiedergegeben. Die in WO 2020/074130 A1 vorgestellten Rahmen für Herzklappenprothesen zum Einsatz bei entzündeten, thrombosierten oder degenerierten Herzklappen besitzen einen zweiteiligen Aufbau, wobei zwischen dem einen, die neuen Herzklappensegel tragenden Teil und dem anderen, als Widerlager fungierenden Teil die infizierten Bereiche kompartimentiert und behandelt werden. Diese Zweiteilige Lösung weist folgende Nachteile auf: Die Einbringung von zwei Teilen ist zeitaufwändiger und technisch kompliziert, da für eine effiziente Kompartimentierung während der kathetergeführten Implantation eine form- und/oder kraftschlüssige Verbindung zwischen beiden Teilen erreicht werden muss. Auch kleinere Lücken zwischen beiden Teilen könnten ein zumindest teilweises Auswaschen der Wirkstoffe im kompartimentierten Vorlumen begünstigen und so eine Behandlung mit ausreichend hohen Konzentrationen gefährden. Weiterhin könnten zweiteilige Lösungen an den Verbindungs- oder Berührungsstellen beider Teile durch die ständige pulsatile Bewegung des schlagenden Herzens Abrieberscheinungen zeigen, die zu Materialermüdung oder Embolisationen führen könnten.

Das Dokument US 2011/0208298 A1 betrifft eine Vorrichtung, die eine Mitralklappenprothese zur Implantation in einem nativen Mitralklappenkomplex eines Patienten umfasst. Die Prothese umfasst eine innere Stützstruktur mit einem stromabwärts gelegenen Abschnitt und einem stromaufwärts gelegenen Abschnitt, wobei der stromaufwärts gelegene Abschnitt eine größere Querschnittsfläche als der stromabwärts gelegene Abschnitt aufweist, und wobei die innere Stützstruktur so konfiguriert ist, dass sie zumindest teilweise auf einer Vorhofseite des nativen Klappenkomplexes positioniert werden kann und eine axiale Kraft in Richtung eines linken Ventrikels ausüben kann. Die Prothese umfasst ferner eine äußere Stützstruktur mit zwei oder mehr Eingriffsarmen, wobei die Eingriffsarme mit der inneren Stützstruktur verbunden sind. Die Prothese ist so konfiguriert, dass sie nach ihrer Implantation Teile der Segel der natürlichen Klappe zwischen der inneren Stützstruktur und den Eingriffsarmen einklemmt.

### Grundsätzliche technische Lösung des Problems

Die Erfindung löst das o.g. klinische Problem, indem kathetergeführte Herzklappenprothesen so konstruiert sind, dass die infizierten Bereiche mittels Kompartimentierungsclips vom Blutstrom abgetrennt werden und auf der Herzklappenprothese wirkstofffreisetzende Matrices angebracht sind, die die Entzündung durch Kompartimentierung lokal therapieren (Wirkstoff-freisetzende Herzklappenprothese). Darüber hinaus ist die Herzklappenprothese so konstruiert, dass infektiöse Auflagerungen (Vegetationen) mit den Kompartimentierungsclips und daran befestigten Strukturen umgriffen und somit eine Embolisation verhindert wird. Ausführungsformen dieser kathetergeführten Herzklappenkonstruktionen erlauben auch einer insgesamt seltenen (1 - 1,5% im ersten Jahr postoperativ) auftretende postoperative PVE vorzubeugen.

Die Erfindung betrifft eine Verankerung für Herzklappenprothesen gemäß dem unabhängigen Patentanspruch 1 sowie einen Katheter zum Einführen einer Verankerung für Herzklappenprothesen in den Körper eines Patienten gemäß dem unabhängigen Patentanspruch 8 und eine Verankerung für Herzklappenprothesen gemäß dem unabhängigen Patentanspruch 11, wobei vorteilhafte Weiterbildungen der Erfindung in den entsprechenden abhängigen Ansprüchen angegeben sind.

Nach einem ersten Aspekt betrifft die Erfindung eine Verankerung für Herzklappenprothesen, insbesondere für Herzklappenprothesen zur Behandlung, Prävention, und/oder zum Ersatz einer Herzklappe, insbesondere einer entzündeten, infizierten, thrombosierten und/oder degenerierten Herzklappe, wobei die Verankerung eine Grundform aufweist und zum minimal-invasiven Einführen der Verankerung in den Körper des Patienten reversibel und insbesondere durch elastische Umformung und/oder Kompression zumindest von Bereichen oder Komponenten der Verankerung in eine Zuführungsform überführbar ist, wobei die Verankerung folgendes aufweist: ein Stentgerüst, welches dazu ausgebildet ist, mindestens zwei Herzklappensegel an einer Innenseite zu befestigen, wobei das Stentgerüst ein proximales Ende und ein distales Ende aufweist; und mindestens zwei Kompartimentierungsclips, welche jeweils an einem ersten Endbereich mit dem Stentgerüst verbunden sind und einen freien, zweiten Endbereich aufweisen, wobei in der Grundform der Verankerung die Kompartimentierungsclips eine Bogenform zwischen den ersten und zweiten Endbereichen aufweisen.

Nach einer weiteren Ausführungsform überlappen, in der Grundform der Verankerung, die Kompartimentierungsclips das Stentgerüst zumindest teilweise überlappen, insbesondere in radialer Richtung des Stentgerüsts.

Gemäß der Erfindung sind die Kompartimentierungsclips derart elastisch umklappbar, dass sich in der Zuführungsform der Verankerung die Kompartimentierungsclips zumindest im Wesentlichen flach zwischen den ersten und zweiten Endbereichen erstrecken.

Nach einer weiteren Ausführungsform weisen die Kompartimentierungsclips Arme auf, welche in der Grundform der Verankerung zumindest bereichsweise verdrillt sind.

Nach einer weiteren Ausführungsform weisen die Kompartimentierungsclips eine erste Clipoberfläche und eine der ersten Clipoberfläche gegenüberliegende zweite Clipoberfläche auf, wobei sowohl in der Grundform als auch in der Zuführungsform der Verankerung die erste Clipoberfläche am freien, zweiten Endbereich der Kompartimentierungsclips zumindest im Wesentlichen radial nach innen, insbesondere in Richtung einer Längsachse der Verankerung, gerichtet ist.

Nach einer weiteren Ausführungsform ist sowohl in der Grundform als auch in der Zuführungsform der Verankerung die zweite Oberfläche am zweiten Endbereich der Kompartimentierungsclips zumindest im Wesentlichen radial nach außen gerichtet.

Nach einer weiteren Ausführungsform ist die Verankerung dazu ausgebildet ist, in einem implantierten Zustand der Verankerung native Segel bzw. Leaflets und/oder infektiöse und/oder thrombotische Auflagerungen/Vegetationen der erkrankten Herzklappe zwischen dem Stentgerüst und den Kompartimentierungsclips zu halten, insbesondere einzuklemmen.

Gemäß der Erfindung weist die Verankerung eine Trennstruktur auf, die derart mit dem Stentgerüst und den Kompartimentierungsclips verbunden ist, dass im implantierten Zustand der Verankerung die Trennstruktur die zu behandelnden Bereiche der nativen Segel bzw. Leaflets vom Blutstrom trennt.

Nach einer weiteren Ausführungsform weist die Trennstruktur eine erste Strukturoberfläche, welche in der Zuführungsform der Verankerung radial nach innen, insbesondere in Richtung einer Längsachse der Verankerung, gerichtet ist, und eine der ersten Strukturoberfläche gegenüberliegende, zweite Strukturoberfläche auf, und wobei zumindest die zweite Strukturoberfläche eine Matrix aufweist, welche antibiotische, antithrombotische und/oder thrombolytische Wirkstoffe enthält und abgeben kann.

Nach einer weiteren Ausführungsform sind das Stentgerüst und die Kompartimentierungsclips aus einem Stück gefertigt.

Nach einer weiteren Ausführungsform sind das Stentgerüst und/oder die Kompartimentierungsclips aus einem komprimierbaren Werkstoff gefertigt.

Nach einer weiteren Ausführungsform sind das Stentgerüst und/oder die Kompartimentierungsclips aus einem selbstexpandierenden Werkstoff gefertigt.

Nach einer weiteren Ausführungsform sind das Stentgerüst und die Kompartimentierungsclips aus mindestens zwei miteinander verbundenen oder verbindbaren Stücken eines selbstexpandierbaren Werkstoffs gefertigt.

Nach einer weiteren Ausführungsform sind das Stentgerüst und die Kompartimentierungsclips aus mindestens zwei miteinander verbundenen oder verbindbaren Stücken gefertigt, wobei das Stentgerüst aus einem ballonexpandierbaren Werkstoff und die Kompartimentierungsclips aus einem selbstexpandierbaren Werkstoff gefertigt sind.

Nach einer weiteren Ausführungsform bestehen das Stentgerüst und/oder die Kompartimentierungsclips aus einer Formgedächtnislegierung, insbesondere Nitinol.

Nach einer weiteren Ausführungsform überlappen in der Grundform der Verankerung die mindestens zwei Kompartimentierungsclips das Stentgerüst derart, dass kein Teil der Kompartimentierungsclips über das distale Ende des Stentgerüsts hinausragt.

Nach einem weiteren Aspekt betrifft die Erfindung einen Katheter nach Anspruch 8 zum Einführen einer Verankerung für Herzklappenprothesen in den Körper eines Patienten, wobei mit Hilfe des Katheters die Verankerung in einer Zuführungsform minimal-invasiv in den Körper des Patienten einführbar und im Implantationsort an der erkrankten Herzklappe in einen implantierten Zustand überführbar ist, und wobei die Verankerung folgendes aufweist:
- ein Stentgerüst welches dazu ausgebildet ist, mindestens zwei Herzklappensegel oder Leaflets an einer Innenseite zu befestigen, wobei das Stentgerüst ein proximales Ende und ein distales Ende aufweist;
- mindestens zwei Kompartimentierungsclips, welche mit dem Stentgerüst verbunden sind, und welche in einer Grundform der Verankerung das Stentgerüst zumindest teilweise überlappen, um native Segel bzw. Leaflets und/oder infektiöse und/oder thrombotische Auflagerungen/Vegetationen der erkrankten Herzklappe zwischen dem Stentgerüst und den Kompartimentierungsclips zu halten, insbesondere einzuklemmen,
wobei der Katheter eine Katheterspitze aufweist, welche über eine Handhabe des Katheters derart manipulierbar ist, dass das Implantat schrittweise von der Katheterspitze freisetzbar ist.

Nach einer weiteren Ausführungsform ist die Katheterspitze derart geteilt ausgebildet, dass die mindestens zwei Kompartimentierungsclips schrittweise, nacheinander von der Katheterspitze freisetzbar sind, und dann folgend das Stentgerüst freigesetzt werden kann.

Nach einer weiteren Ausführungsform weist die geteilte Katheterspitze eine angeschrägte Hülse auf, welche dazu ausgebildet ist, die Verankerung während des Einbringens zu überdecken, und welche mit Hilfe der Handhabe zurückziehbar ist, um die Kompartimentierungsclips schrittweise freizusetzen.

Nach einer weiteren Ausführungsform ist der Katheter dazu ausgebildet, die Verankerung über die Handhabe zu rotieren, um Kompartimentierungsclips auf die Taschen/Segel/Leaflets der nativen Klappe auszurichten.

Nach einem weiteren Aspekt betrifft die Erfindung ein System nach Anspruch 10 zur Behandlung, Prävention und/oder zum Ersatz einer Herzklappe, insbesondere einer entzündeten, infizierten, thrombosierten oder degenerierten Herzklappe, wobei das System eine der oben stehenden Verankerung und einen der oben stehenden Katheter nach aufweist, wobei die Verankerung dazu ausgebildet ist, insbesondere in der Zuführungsform der Verankerung in der Katheterspitze aufnehmbar zu sein, und wobei die Katheterspitze ausgebildet ist, die Verankerung, insbesondere in der Zuführungsform der Verankerung, aufzunehmen.

Bei einem Verfahren zur Behandlung, Prävention, und/oder zum Ersatz einer Herzklappe, insbesondere einer entzündeten, infizierten, thrombosierten oder degenerierten Herzklappe, weist das Verfahren folgendes auf:
- Einführen einer Verankerung mit Hilfe eines Katheters;
- Freisetzen der Kompartimentierungsclips;
- Ausrichten der Kompartimentierungsclips auf einer ersten Seite der nativen Segel/Leaflets der erkrankten Herzklappe;
- Freisetzen des Stentgerüsts auf einer zweiten Seite der nativen Segel, welche der ersten Seite gegenüberliegt.

Nach einer Ausführungsform weist das Verfahren ferner den Verfahrensschritt der Freigabe von antibiotischen, antithrombotischen, thrombolytischen und/oder zellwachstumshemmenden Wirkstoffen auf, wobei die Freigabe in-situ erfolgt.

Nach einer weiteren Ausführungsform werden die Kompartimentierungsclips schrittweise, nacheinander freigesetzt werden.

Offenbart ist ferner eine nicht beanspruchte Verankerung für Herzklappenprothesen, insbesondere für Herzklappenprothesen zur Behandlung, Prävention, und/oder zum Ersatz einer Herzklappe, insbesondere einer entzündeten, infizierten, thrombosierten oder degenerierten Herzklappe, wobei die Verankerung eine Grundform aufweist, und wobei die Verankerung zum minimal-invasiven Einführen der Verankerung in den Körper des Patienten reversibel und insbesondere durch elastische Umformung und/oder Kompression zumindest von Bereichen oder Komponenten der Verankerung in eine Zuführungsform überführbar ist, wobei die Verankerung folgendes aufweist:
- ein Stentgerüst, welches dazu ausgebildet ist, mindestens zwei Herzklappensegel an einer Innenseite zu befestigen, wobei das Stentgerüst ein proximales Ende und ein distales Ende aufweist;
- mindestens zwei Stabilisierungsbögen, welche über das distale Ende des Stentgerüsts hinausragen und dazu ausgebildet sind, sich im implantierten Zustand der Verankerung derart an einer Gefäßwand abzustützen, dass die Verankerung nach Freisetzung der Stabilisierungsbögen zentral in dem zu implantierenden Blutgefäß ausgerichtet ist.

Nach einer weiteren Ausführungsform (nicht beansprucht) sind die mindestens zwei Stabilisierungsbögen in ihrer Länge und/oder Form so ausgebildet, dass sie im Röntgenbild unterscheidbar sind und bei der Implantation nacheinander freigesetzt werden können.

Nach einer weiteren Ausführungsform (nicht beansprucht) weisen die Stabilisierungsbögen ein proximales Ende, das mit dem distalen Ende des Stentgerüsts verbunden ist, und ein distales Ende, das über distale Ende des Stentgerüsts hinausragt, auf, wobei die Stabilisierungsbögen am distalen Ende durch Streben, Rauten oder andere Elemente miteinander verbunden sind, welche dazu ausgebildet sind, die radiale Stabilität zu vergrößern und eine Zentralisierung der Verankerung im zu implantierbaren Blutgefäß zu ermöglichen.

Nach einer weiteren Ausführungsform weist die Verankerung eine Trennstruktur auf, die derart mit dem Stentgerüst verbunden ist, dass die Trennstruktur im expandierten Zustand der Verankerung die zu behandelnden Bereiche der nativen Segel bzw. Leaflets vom Blutstrom trennt.

Nach einer weiteren Ausführungsform weist die Trennstruktur zumindest außenseitig eine Matrix auf, welche antibiotische, antithrombotische und/oder thrombolytische Wirkstoffe enthält und abgeben kann.

Nach einer weiteren Ausführungsform (nicht beansprucht) sind das Stentgerüst und die Stabilisierungsbögen aus einem Stück gefertigt.

Nach einer weiteren Ausführungsform (nicht beansprucht) sind das Stentgerüst und/oder die Stabilisierungsbögen aus einem komprimierbaren Werkstoff gefertigt.

Nach einer weiteren Ausführungsform (nicht beansprucht) sind das Stentgerüst und/oder die Stabilisierungsbögen aus einem selbstexpandierenden Werkstoff gefertigt sind.

Nach einer weiteren Ausführungsform (nicht beansprucht)bestehen das Stentgerüst und/oder die Stabilisierungsbögen aus einer Formgedächtnislegierung, insbesondere Nitinol.

Nach einer weiteren Ausführungsform weist die Verankerung mindestens zwei Kompartimentierungsclips, welche jeweils an einem ersten Endbereich mit dem Stentgerüst verbunden sind und einen freien, zweiten Endbereich aufweisen, wobei die Kompartimentierungsclips in der Grundform der Verankerung eine Bogenform zwischen den ersten und zweiten Endbereichen aufweisen.

Nach einer weiteren Ausführungsform überlappen die Kompartimentierungsclips in der Grundform der Verankerung das Stentgerüst zumindest teilweise.

Nach einer weiteren Ausführungsform sind die Kompartimentierungsclips derart umklappbar, dass sich die Kompartimentierungsclips in der Zuführungsform der Verankerung zumindest im Wesentlichen flach zwischen den ersten und zweiten Endbereichen erstrecken.

Nach einem weiteren Aspekt betrifft die Erfindung gemäß Anspruch 11 eine Verankerung für Herzklappenprothesen, insbesondere für Herzklappenprothesen zur Behandlung, Prävention, und/oder zum Ersatz einer Herzklappe, insbesondere einer entzündeten, infizierten, thrombosierten oder degenerierten Herzklappe, wobei die Verankerung eine Grundform aufweist, und wobei zum minimal-invasiven Einführen der Verankerung in den Körper des Patienten die Verankerung reversibel und insbesondere durch elastische Umformung und/oder Kompression zumindest von Bereichen oder Komponenten der Verankerung in eine Zuführungsform überführbar ist, wobei die Verankerung folgendes aufweist:
- ein Stentgerüst welches dazu ausgebildet ist, mindestens zwei Herzklappensegel bzw. Leaflets an einer Innenseite zu befestigen, wobei das Stentgerüst ein proximales Ende und ein distales Ende aufweist;
- mindestens zwei Kompartimentierungsclips, welche mit dem Stentgerüst verbunden sind und, in der Grundform der Verankerung, das Stentgerüst zumindest teilweise überlappen, um native Segel und/oder infektiöse und/oder thrombotische Auflagerungen/Vegetationen der erkrankten Herzklappe, zwischen dem Stentgerüst und den Kompartimentierungsclips zu halten, insbesondere einzuklemmen;
- eine Trennstruktur, die derart mit dem Stentgerüst und den Kompartimentierungsclips verbunden ist, dass die Trennstruktur, im implantierten Zustand, die zu behandelnden Bereiche der nativen Segel vom Blutstrom trennt.

Nach einer weiteren Ausführungsform weist die Trennstruktur eine erste Strukturoberfläche, welche in der Zuführungsform radial nach innen, insbesondere in Richtung einer Längsachse der Verankerung, gerichtet ist, und eine der ersten Strukturoberfläche gegenüberliegende, zweite Strukturoberfläche auf, wobei zumindest die zweite Strukturoberfläche eine Matrix aufweist, welche antibiotische, antithrombotische und/oder thrombolytische Wirkstoffe enthält und abgeben kann.

Nach einer weiteren Ausführungsform ist die mindestens eine wirkstoffhaltige Matrix bioresorbierbar ist.

Nach einer weiteren Ausführungsform sind das Stentgerüst und die Kompartimentierungsclips aus einem Stück gefertigt.

Nach einer weiteren Ausführungsform sind das Stentgerüst und/oder die Kompartimentierungsclips aus einem komprimierbaren Werkstoff gefertigt.

Nach einer weiteren Ausführungsform sind das Stentgerüst und/oder die Kompartimentierungsclips aus einem selbstexpandierbaren Werkstoff gefertigt.

Nach einer weiteren Ausführungsform sind das Stentgerüst und die Kompartimentierungsclips aus mindestens zwei miteinander verbundenen oder verbindbaren Stücken eines selbstexpandierbaren Werkstoffs gefertigt.

Nach einer weiteren Ausführungsform sind das Stentgerüst und die Kompartimentierungsclips aus mindestens zwei miteinander verbundenen verbindbaren Stücken gefertigt sind, und das Stentgerüst aus einem ballonexpandierbaren Werkstoff und die Kompartimentierungsclips aus einem selbstexpandierbaren Werkstoff gefertigt.

Nach einer weiteren Ausführungsform bestehen das Stentgerüst und/oder die Kompartimentierungsclips aus einer Formgedächtnislegierung, insbesondere Nitinol.

Gemäß der Erfindung weisen die mindestens zwei Kompartimentierungsclips an einen ersten Endbereich, der mit dem Stentgerüst verbunden ist, und einen freien, zweiten Endbereich auf, wobei die Kompartimentierungsclips in der Grundform der Verankerung eine Bogenform zwischen den ersten und zweiten Endbereichen aufweisen.

Nach einer weiteren Ausführungsform überlappen die Kompartimentierungsclips, in der Grundform der Verankerung, das Stentgerüst zumindest teilweise.

Nach einer weiteren Ausführungsform sind die Kompartimentierungsclips derart umklappbar, dass sich die Kompartimentierungsclips in der Zuführungsform der Verankerung zumindest im Wesentlichen flach zwischen den ersten und zweiten Endbereichen erstrecken.

Nach einer weiteren Ausführungsform weist die Verankerung drei Kompartimentierungsclips auf.

Nach einer weiteren Ausführungsform sind die mindestens zwei Kompartimentierungsclips im expandierten Zustand der Verankerung radial voneinander beabstandet und über das Stentgerüst miteinander verbunden.

Nach einer weiteren Ausführungsform weist das Stentgerüst an seinem proximalen Ende eine erste Zellstruktur aus Streben und an seinem distalen Ende eine zweite Zellstruktur aus Streben auf, wobei die erste und die zweite Zellstruktur unterschiedlich ausgebildet sind.

Nach einer weiteren Ausführungsform weist die erste Zellstruktur eine höhere Dichte an Streben auf als die zweite Zellstruktur.

Nach einer weiteren Ausführungsform ist die Verankerung aus einem Rohr aus Formgedächtnislegierung geschnitten.

Nach einem weiteren Aspekt betrifft die Erfindung eine nicht beanspruchte Herzklappenprothese, insbesondere für Herzklappenprothesen zur Behandlung, Prävention, und/oder zum Ersatz einer Herzklappe, insbesondere einer entzündeten, infizierten, thrombosierten oder degenerierten Herzklappe, wobei die Herzklappenprothese folgendes aufweist:
- eine oben beschriebene Verankerung;
- mindestens zwei Herzklappensegel, welche an der Innenseite des Stentgerüsts der Verankerung befestigt sind.

Die mit der Erfindung vorgestellten technischen Umsetzungen beinhaltet folgende Neuheiten und bietet folgende Vorteile gegenüber herkömmlichen kathetergeführten Herzklappenprothesen:
Der kathetergeführte Herzklappenersatz wird auch bei Patienten möglich, die eine bakterielle Endokarditis haben. Dieser weitgehend atraumatische Eingriff kann mit geringeren Risiken bei Patienten mit hoher Komorbidität, durchgeführt werden. Die durch die lokale Applikation erreichbaren antibakteriellen Wirkstoffkonzentrationen im Gewebe, sind deutlich höher, als die durch systemische Gaben erzielt werden kann. Systemische Antibiotikagaben sind begrenzt durch toxische Arzneimittelnebenwirkungen.

Ausführungsformen der erfindungsgemäßen Konstruktion einer solchen kathetergeführten Herzklappenprothese können das Risiko einer postoperativen Endokarditis (siehe oben) auf Grund einer prophylaktischen Langzeitantibiotikaabgabe reduzieren, indem die Besiedlung der Herzklappenprothese mit Mikroorganismen, insbesondere mit Bakterien, während der Phase des Einheilens, vor einer Infektion geschützt ist.

Die erfindungsgemäßen Konstruktionen der Herzklappenprothese erlaubt auch die Behandlung einer Herzklappenundichtigkeit (z.B. Aortenklappeninsuffizienz) als auch einer Herzklappenverengung (Stenose), indem die Fixation des Herzklappenimplantats über Kompartimentierungsclips an den nativen Herzklappensegeln erfolgt.

Die Erfindung erweitert somit das gegenwärtige Indikationsspektrum der kathetergeführten Herzklappenoperation entscheidend, indem die Therapie und Prophylaxe einer infektiösen Endokarditis (sowohl einer NVE als auch einer PVE) möglich wird.

Eine wesentliche Neuerung für zur Therapie und/oder Prophylaxe der Endokarditis bestimmten Herzklappenprothesen im Vergleich zu Dokument WO 2020/074130 A1 ist, dass die hier vorgestellte erfindungsgemäße Herzklappenprothese aus nur einem Teil aufgebaut ist. Dadurch werden folgende Vorteile erreicht: Die Herzklappenprothesen können in einem Schritt und somit mit weniger Zeit- und Materialaufwand implantiert werden, weil nicht zwei Teile form- oder kraftschlüssig gegeneinander ausgerichtet werden müssen. Weiterhin ergibt sich nicht das Problem, dass in der gesamten Zirkumferenz an den Verbindungs- bzw. Kontaktstellen mit den beschriebenen Problemen entstehen. Die erfindungsgemäßen einteiligen Herzklappenprothesen schaffen eine lückenlose Verbindung der beiden Bereiche, die innerhalb (luminal) und außerhalb (abluminal) der infizierten und ggf. mit infektiösen Auflagerungen (Vegetationen) belegten nativen Herzklappensegel umfassen und kompartimentieren. Die Kompartimentierung wird dadurch verbessert und es können bei den hier vorgestellten einteiligen Herzklappenprothesen höhere lokale Wirkstoffkonzentrationen erreicht werden.

Verschiedene Ausführungsformen der erfindungsgemäßen Herzklappe enthalten stets ein einziges zu implantierendes Metallgerüst, wobei dieses eine Gerüst (z.B. bei der Herstellung) auch aus mehreren Teilen zusammengesetzt sein kann, um beispielsweise verschiedene Materialien, um bestimmte Materialeigenschaften (selbstexpandierbar, ballonexpandierbar) zu kombinieren und/oder um bestimmte Stabilitäten oder Formgebungen umzusetzen. Für die Verbindung der einzelnen Teile zu dem einen Metallgerüst können verschiedenste Verfahren, u.a. Schweißen, Kleben, Nähen, Stecken etc. zum Einsatz kommen. Neben der Möglichkeit, dieses eine Metallgerüst auch aus verschiedenen Teilen zusammenzusetzen, sind ausdrücklich auch Ausführungsformen erwähnt, die aus einem einzigen Material und einem einzigen Stück gefertigt sind (beispielsweise ein aus einem einzigen Rohr einer Formgedächtnislegierung laser-geschnittenes Werkstück, dass in einem nachfolgenden Formgebungsprozess in Form gebracht wird).

Bei der kathethergeführten Implantation von Herzklappenprothesen - insbesondere, wenn wie z.B. bei einem transfemoralen Einführen des Katheters ins Blutsystem die Herzklappenprothese einen längeren Weg durch die Blutgefäße bis zum Implantationsort vorgeschoben werden muss - ist es auf Grund der begrenzten anatomischen Verhältnisse vorteilhaft, wenn die Herzklappenprothesen zur Implantation auf einen möglichst kleinen Durchmesser komprimiert ("gecrimpt") werden können. Würden die Strukturen und Materialien von zweiteiligen Herzklappenprothesen (Stentgerüst und Widerlagerstruktur) für den Ersatz von infizierten Herzklappen lediglich in einem Teil kombiniert werden, so würde die resultierende einteilige Lösung konsequenterweise auch im komprimierten/gecrimpten Zustand eine deutliche Umfangszunahme erfahren. Die hier vorgestellten erfindungsgemäßen einteiligen Herzklappenprothesen sind jedoch so aufgebaut, dass die Herzklappenprothese speziell konstruierte Kompartimentierungsclips enthält, welche bei Aufnahme in den Katheter also vor der Implantation, in longitudinaler (axialer) Richtung nach oben gebogen und zusammen mit dem Stentgerüst komprimiert werden können und so das Material nicht auf einer axialen Höhe übereinanderliegt, sondern über die axiale Länge verteilt wird. Dies ermöglicht die Einbringung des Einteilers mit gängigen Katheterdurchmessern. Während der Implantation werden die nach oben gebogenen Kompartimentierungsclips nach unten entlassen, wobei diese Freisetzung schrittweise erfolgen kann. Eine schrittweise Freisetzung kann erforderlich sein, da das Umklappen der (ggf. z.Z. noch komprimierten) bogenartigen Kompartimentierungsclips mit einer vorübergehenden Umfangszunahme einhergeht.

Neben dem Umklappen der Kompartimentierungsbögen können die Bögen in anderen erfindungsgemäßen Ausführungsformen auch schrittweise nach unten geschoben werden, sodass keine starke Umfangszunahme während dieses Schrittes des nach-unten-Ausrichtens erfolgt.

Bei der Implantation können mit den nach unten ausgerichteten Kompartimentierungsbögen, die mit einer durchgehenden Trennschicht und ggf. mit einer wirkstoffhaltigen Matrix verbunden sind, beim nach-unten-Schieben der teilweise noch komprimierten Herzklappenprothese die an den nativen Herzklappensegeln anhaftenden/aufgelagerten infektiösen Auflagerungen (Vegetationen) zusammen mit den infizierten Herzklappensegeln selbst umgreifen und einfangen. Dabei werden die Bögen der Kompartimentierungsclips bis in die Taschen der Herzklappen, die von den nativen Herzklappensegeln mit der Gefäßwand gebildet werden, eingeführt. Beim nachfolgenden Expandieren und Freisetzen des Stentgerüsts wird der umgriffene Bereich weiter komprimiert und die umgriffenen Bereiche werden so kompartimentiert. Die mindestens eine wirkstoffhaltige Matrix setzt in diesen kompartimentierten Bereich antibiotische Wirkstoffe frei, um die Infektion lokal in diesem Bereich zu behandeln.

In Ausführungsformen der erfindungsgemäßen Herzklappenprothese sind die Kompartimentierungsclips so angebracht, dass die Bögen (z.B. bei, Schneiden aus einem Materialstück) nicht auf den "Spitzen", sondern in den "Tälern" von rautenartigen Strukturen entspringen und nach oben weisen und dann durch die im Formgebungsprozess eingeprägte Form im expandierten Zustand fast kreisförmig und näherungsweise tangential nach außen weisen und dann an der Peripherie des Stentgerüstes nach unten laufend einen Bogen bilden. Dabei wird den Bögen eine 360°-Torsion eingeprägt, die ermöglicht, dass das Material der Bögen bei dem Prozess des nach oben Biegens vor der Implantation sowie während des nach unten Freisetzens bei der Implantation die auftretenden Verformungen und die damit einhergehenden Kräfte und Spannungen aufnimmt ohne zu brechen oder plastisch zu verformen.

In anderen Ausführungsformen der erfindungsgemäßen Herzklappenprothese reichen die Schenkel der Bögen der Kompartimentierungsclips nach dem Formgebungsprozess weit nach oben und sind dann über einen geeigneten Krümmungsradius nach außen oder näherungsweise tangential im 180° nach unten umgeklappt wobei das unterste Stück für die Implantation wieder nach oben geklappt wird, um diesen Bereich der Kompartimentierungsclips während der Implantation nicht auf gleicher axialer Höhe wie die innerhalb des Stentgerüsts angebrachten Herzklappensegel zu positionieren (Umfangsreduzierung während der Implantation).

Weiterhin können an Ausführungsbeispielen der erfindungsgemäßen Herzklappenprothese nach oben (axial) ausgerichtete Stabilisierungsbögen befestigt sein, welche bei der Implantation freigesetzt werden und in mindestens teilweise expandiertem Zustand die Herzklappenprothese in dem zu implantierenden Gefäß (zentral) positionieren, sodass die Bögen der Kompartimentierungsclips in alle nativen Herzklappensegel geführt werden können. In Ausführungsbeispielen der erfindungsgemäßen Herzklappenprothese können diese Zentralisierungsbögen im axial oberen Bereich durch Bogen-, Rauten- oder andersartige Elemente verbunden sein, um diesen Bereich der Herzklappenprothese mit einer größeren Radialkraft auszustatten. Zudem sind die erfindungsgemäßen Herzklappenprothesen zusammen mit dem Einführkathetersystem so ausgebildet, dass während der Implantation eine axiale Drehung der Herzklappenprothese möglich ist, um die Bögen der Kompartimentierungsclips in die nativen Herzklappensegel einzuführen.

### Kurze Beschreibung der Zeichnungen

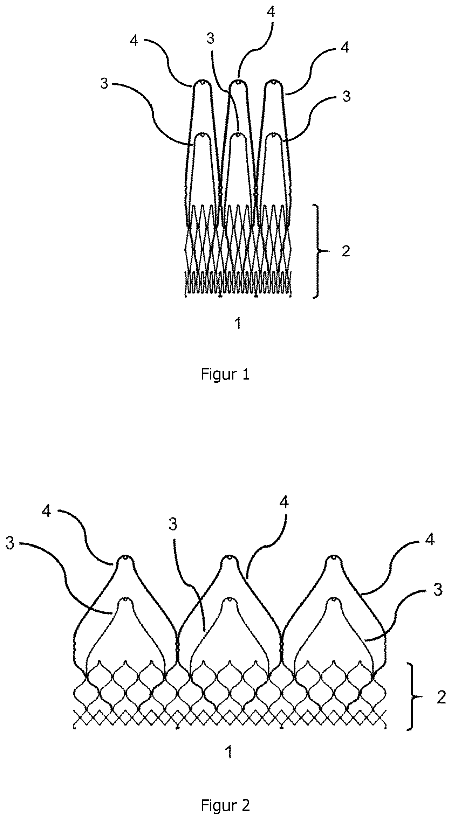

Figur 1 zeigt den geschnittenen Metallrahmen eines Ausführungsbeispiels mit dreizähliger Rotationssymmetrie der Herzklappenprothese zum Einsatz in einer dreisegeligen Herzklappe. Dargestellt ist das auf zwei Dimensionen gebrachte Schnittmuster für einen rohrförmigen Werkstoff.
Figur 2 zeigt den Rahmen der Herzklappenprothese des Ausführungsbeispiels aus Figur 1 in einer zweidimensionalen Darstellung, nachdem es im Laufe des Formgebungsprozesses radial aufgeweitet wurde und die Kompartimentierungsclips nach unten geklappt sind.

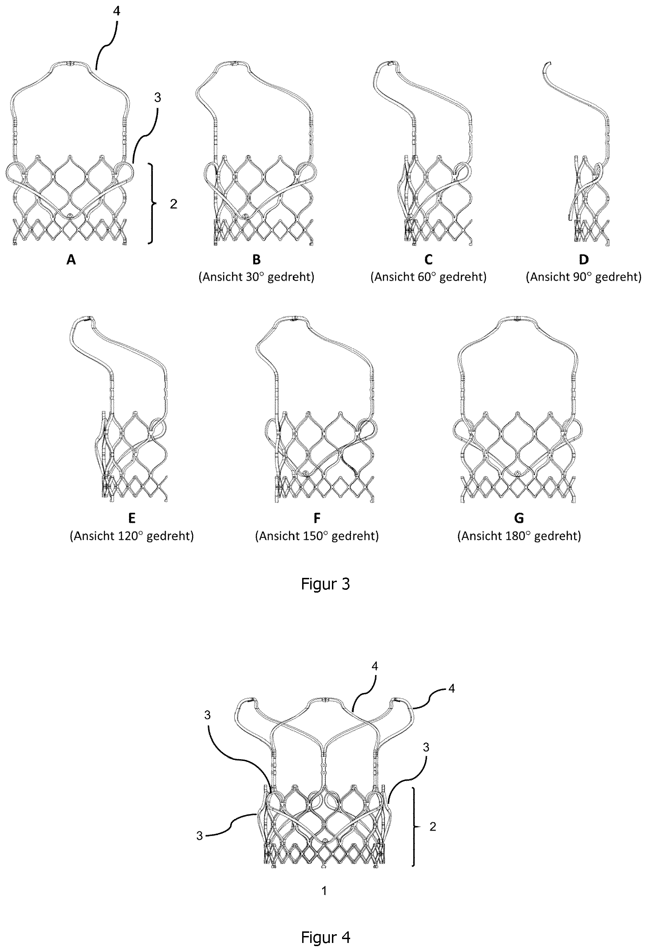

Figur 3 zeigt ein Drittel des Rahmens der Herzklappenprothese des Ausführungsbeispiels aus Figur 1, nach dem Formgebungsprozess, wobei die einzelnen Teile der Figur (A bis G) jeweils dasselbe Teil darstellen und die Ansicht in jeder Teilabbildung um 30° weiter in axialer Richtung gedreht ist.
Figur 4 zeigt eine seitliche Ansicht des Rahmens der Herzklappenprothese eines Ausführungsbeispiels aus Figur 1.

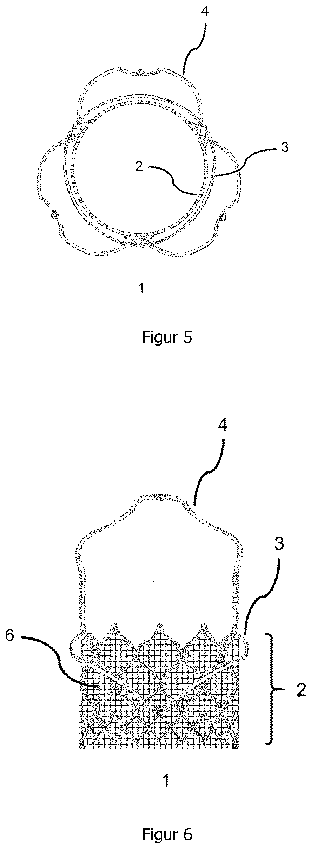

Figur 5 zeigt die Aufsicht auf den Rahmen der Herzklappenprothese eines Ausführungsbeispiels.
Figur 6 zeigt ein Drittel eines Ausführungsbeispiels, in dem der untere Teil des Stentgerüsts, nicht aber die Kompartimentierungsclips mit einer dünnen Schicht zur Abgrenzung gegen den Blutstrom und/oder die wirkstoffhaltigen Bereiche angebracht sind.

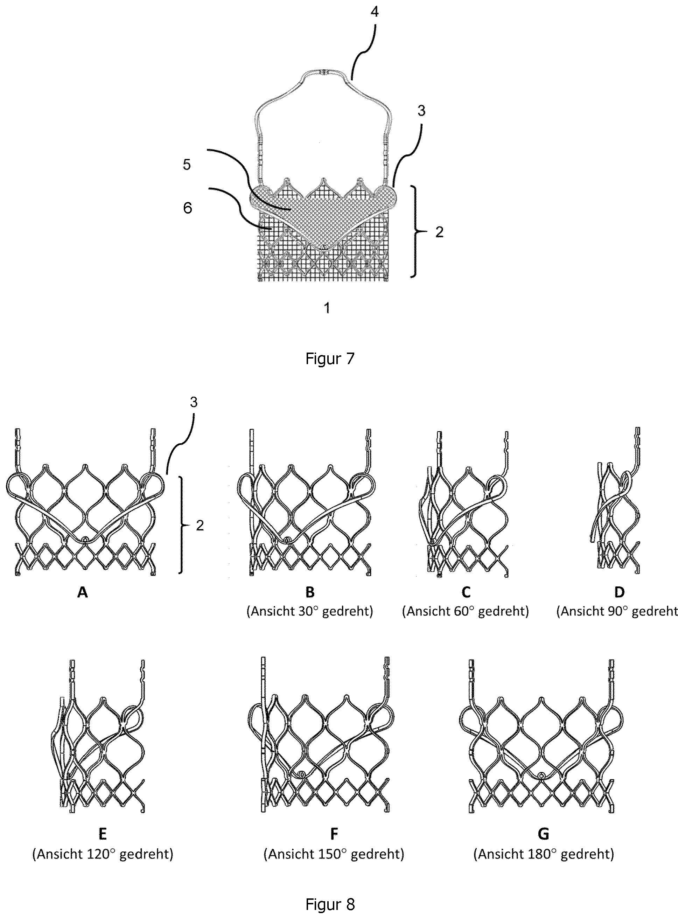

Figur 7 zeigt ein Drittel eines Ausführungsbeispiels, in dem sowohl der untere Teil des Stentgerüsts, als auch die Kompartimentierungsclips flächig mit einer dünnen Schicht zur Abgrenzung gegen den Blutstrom und/oder die wirkstoffhaltigen Bereiche angebracht sind.
Figur 8 zeigt ein Drittel eines weiteren Ausführungsbeispiels des Gerüsts, wobei die das Gerüst der Herzklappenprothese ohne Stabilisierungsbögen ausgebildet ist.

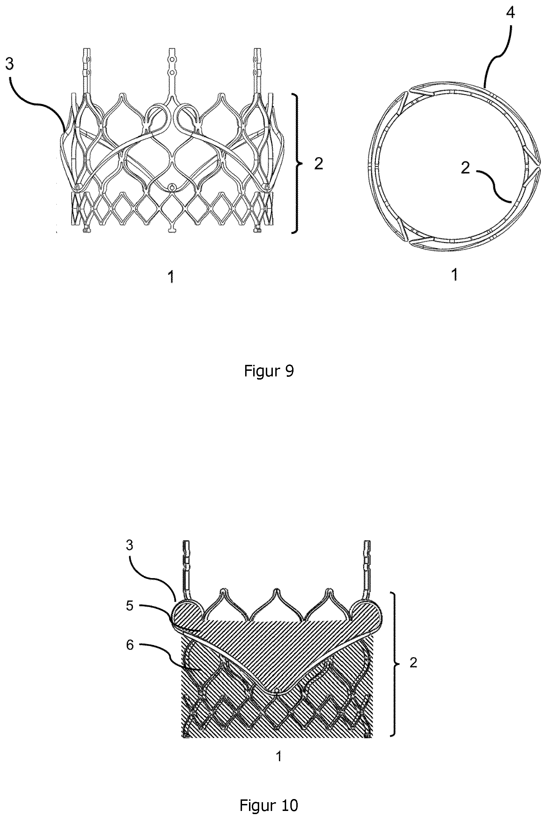

Figur 9 zeigt das gesamte Gerüst des Ausführungsbeispiels aus Figur 8, wobei eine Ansicht von vorne (frontal) und eine Aufsicht von oben (apikal) dargestellt ist.
Figur 10 zeigt einen Teil des Ausführungsbeispiels aus Figuren 8 und 9 mit der Trennschicht und den wirkstofffreisetzenden Matrices, die beide durchgehend am Stentgerüst beginnend bis über die Bögen der Kompartimentierungsclips reichen.

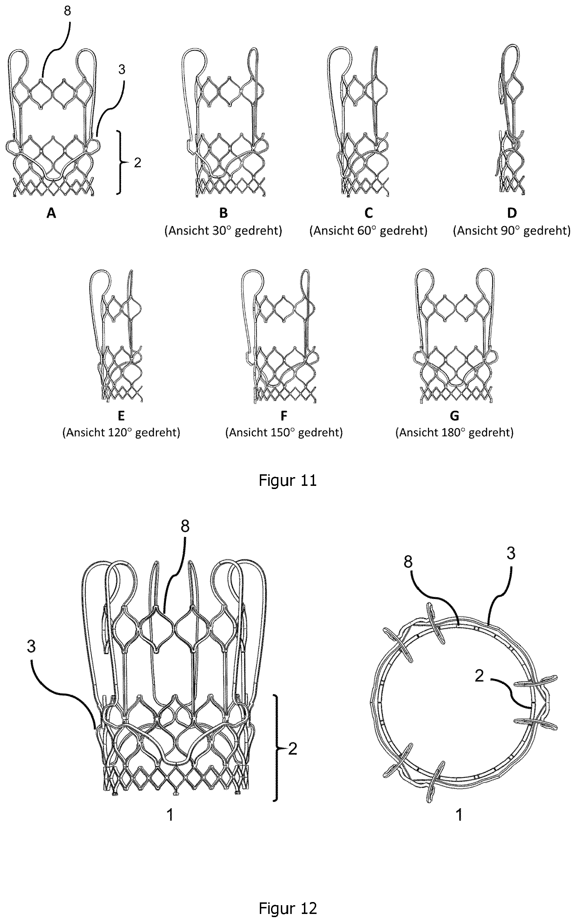

Figur 11 zeigt ein Drittel eines weiteren Ausführungsbeispiels des Gerüsts im expandierten und umgeklappten Zustand, wobei die Kompartimentierungsclips so ausgebildet sind, in einem langen Bogen vom Stentgerüst zuerst nach oben zu verlaufen und dann wieder nach unten zu reichen und wobei die vom Stentgerüst beginnende Trennschicht und wirkstofffreisetzenden Matrices nur im unteren Bereich der Kompartimentierungsclips angebracht sind. Weiterhin sind in diesem Ausführungsbeispiel die Bögen im oberen Bereich zur Stabilisierung und Aufbringung von Radialkraft durch Strukturen miteinander verbunden.
Figur 12 zeigt das gesamte Gerüst des Ausführungsbeispiels aus Figur 11, wobei eine Ansicht von vorne (frontal) und eine Aufsicht von oben (apikal) dargestellt ist.

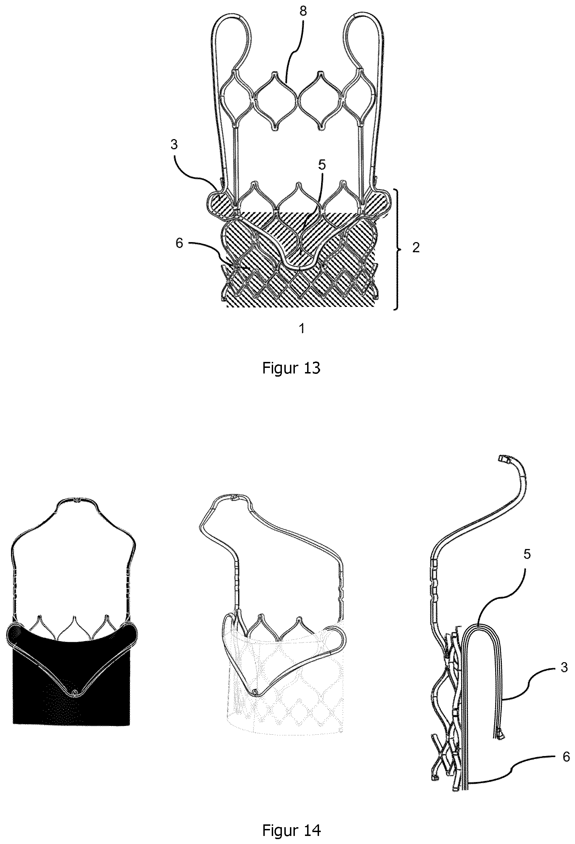

Figur 13 zeigt einen Teil des Ausführungsbeispiels aus Figuren 11 und 12 mit der Trennschicht und den wirkstofffreisetzenden Matrices, die beide durchgehend am Stentgerüst beginnend bis über die Bögen der Kompartimentierungsclips reichen.
Figur 14 zeigt in einem Schnittbild einen schematischen Aufbau der Lage von Stentgerüst mit den Bögen der Kompartimentierungsclips, einer daran befestigten Trennschicht und einer wirkstoffhaltigen Matrix.

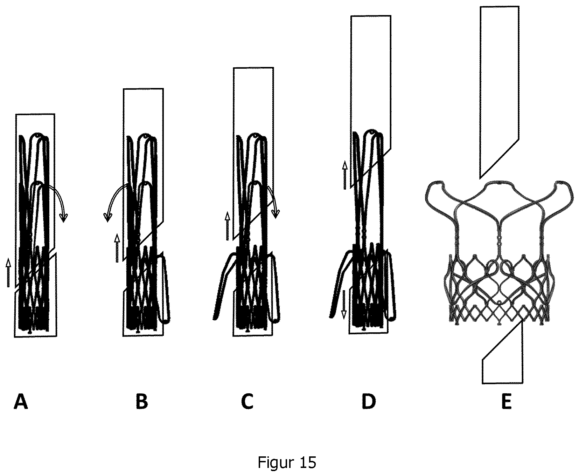

Figur 15 zeigt eine schematische Darstellung der Freisetzung eines Ausführungsbeispiels der erfindungsgemäßen Herzklappenprothese mittels eines an mindestens einem Teil angeschrägten Katheters, wodurch beim Zurückziehen der Katheterhülse bzw. Einführschleuse die Bögen der Kompartimentierungsclips nacheinander freigesetzt werden können.

### Bezugszeichenliste

- 1: Rahmen der Herzklappenprothese
- 2: Stentgerüst
- 3: Kompartimentierungsclips
- 4: Stabilisierungsbögen
- 5: Trennschicht
- 6: Wirkstoffhaltige Bereiche /Matrices

## Patentansprüche

1. Verankerung für Herzklappenprothesen zur Behandlung, Prävention, und/oder zum Ersatz einer Herzklappe, insbesondere einer entzündeten, infizierten, thrombosierten und/oder degenerierten Herzklappe, wobei die Verankerung eine Grundform aufweist und zum minimal-invasiven Einführen der Verankerung in den Körper des Patienten reversibel und durch elastische Umformung und/oder Kompression zumindest von Bereichen oder Komponenten der Verankerung in eine Zuführungsform überführbar ist, wobei die Verankerung folgendes aufweist:
• ein Stentgerüst (2), welches dazu ausgebildet ist, mindestens zwei Herzklappensegel an einer Innenseite zu befestigen, wobei das Stentgerüst ein proximales Ende und ein distales Ende aufweist;
• mindestens zwei Kompartimentierungsclips (3), welche jeweils an einem ersten Endbereich mit dem Stentgerüst verbunden sind und einen freien, zweiten Endbereich aufweisen,
wobei in der Grundform der Verankerung die Kompartimentierungsclips (3) eine Bogenform zwischen den ersten und zweiten Endbereichen aufweisen,
wobei die Kompartimentierungsclips (3) derart elastisch umklappbar sind, dass sich in der Zuführungsform der Verankerung die Kompartimentierungsclips zumindest im Wesentlichen flach zwischen den ersten und zweiten Endbereichen erstrecken,
und wobei die Verankerung eine Trennstruktur aufweist, die derart mit dem Stentgerüst (2) und den Kompartimentierungsclips (3) verbunden ist, dass im implantierten Zustand der Verankerung die Trennstruktur (5) die zu behandelnden Bereiche der nativen Herzklappensegel vom Blutstrom trennt.

2. Verankerung nach Anspruch 1,
wobei in der Grundform der Verankerung die Kompartimentierungsclips (3) das Stentgerüst zumindest teilweise überlappen, insbesondere in radialer und proximaler Richtung des Stentgerüsts.

3. Verankerung nach Anspruch 1 oder 2,
wobei die Kompartimentierungsclips Arme aufweisen, welche in der Grundform der Verankerung zumindest bereichsweise verdrillt sind, wobei vorzugsweise die Kompartimentierungsclips (2) eine erste Clipoberfläche und eine der ersten Clipoberfläche gegenüberliegende zweite Clipoberfläche aufweisen, und wobei sowohl in der Grundform als auch in der Zuführungsform der Verankerung die erste Clipoberfläche am freien, zweiten Endbereich der Kompartimentierungsclips zumindest im Wesentlichen radial nach innen, insbesondere in Richtung einer Längsachse der Verankerung, gerichtet ist.

4. Verankerung nach Anspruch 1 oder 2,
wobei die Kompartimentierungsclips Arme aufweisen, welche in der Grundform der Verankerung zumindest bereichsweise verdrillt sind, wobei vorzugsweise die Kompartimentierungsclips (2) eine erste Clipoberfläche und eine der ersten Clipoberfläche gegenüberliegende zweite Clipoberfläche aufweisen, und wobei in der Grundform die erste Clipoberfläche nach außen und die zweite Clipoberfläche nach innen und in der Zuführungsform die erste Oberfläche nach innen und die zweite Oberfläche nach außen gerichtet ist.

5. Verankerung nach einem der Ansprüche 1 bis 4,
wobei die Verankerung dazu ausgebildet ist, in einem implantierten Zustand der Verankerung native Herzklappensegel und/oder infektiöse und/oder thrombotische Auflagerungen/Vegetationen der erkrankten Herzklappe zwischen dem Stentgerüst und den Kompartimentierungsclips (3) zu halten, insbesondere einzuklemmen; und/oder wobei die Trennstruktur eine erste Strukturoberfläche, welche in der Zuführungsform der Verankerung radial nach innen, insbesondere in Richtung einer Längsachse der Verankerung, gerichtet ist, und eine der ersten Strukturoberfläche gegenüberliegende, zweite Strukturoberfläche aufweist, und wobei zumindest die zweite Strukturoberfläche eine Matrix aufweist, welche antibiotische, antithrombotische und/oder thrombolytische Wirkstoffe enthält und abgeben kann.

6. Verankerung nach einem der Ansprüche 1 bis 5,
wobei das Stentgerüst und die Kompartimentierungsclips aus einem Stück gefertigt sind; oder
wobei das Stentgerüst und/oder die Kompartimentierungsclips aus einem komprimierbaren Werkstoff gefertigt sind; oder
wobei das Stentgerüst und/oder die Kompartimentierungsclips aus einem selbstexpandierenden Werkstoff gefertigt sind; oder
wobei das Stentgerüst und die Kompartimentierungsclips aus mindestens zwei miteinander verbundenen oder verbindbaren Stücken eines selbstexpandierbaren Werkstoffs gefertigt sind; oder
wobei das Stentgerüst und die Kompartimentierungsclips aus mindestens zwei miteinander verbundenen oder verbindbaren Stücken gefertigt sind, und das Stentgerüst aus einem ballonexpandierbaren Werkstoff und die Kompartimentierungsclips aus einem selbstexpandierbaren Werkstoff gefertigt sind; oder
wobei das Stentgerüst und/oder die Kompartimentierungsclips aus einer Formgedächtnislegierung, insbesondere Nitinol, bestehen.

7. Verankerung nach einem der Ansprüche 1 bis 6,
wobei in der Grundform der Verankerung die mindestens zwei Kompartimentierungsclips (3) das Stentgerüst (2) derart überlappen, dass kein Teil der Kompartimentierungsclips über das distale Ende des Stentgerüsts (2) hinausragt.

8. Katheter zum Einführen einer Verankerung für Herzklappenprothesen in den Körper eines Patienten, wobei mit Hilfe des Katheters die Verankerung in einer Zuführungsform minimal-invasiv in den Körper des Patienten einführbar und im Implantationsort an der erkrankten Herzklappe in einen explantierten Zustand überführbar ist, und wobei die Verankerung folgendes aufweist:
• ein Stentgerüst (2) welches dazu ausgebildet ist, mindestens zwei Herzklappensegel oder Leaflets an einer Innenseite zu befestigen, wobei das Stentgerüst ein proximales Ende und ein distales Ende aufweist;
• mindestens zwei Kompartimentierungsclips (3), welche mit dem Stentgerüst verbunden sind, und welche in einer Grundform der Verankerung das Stentgerüst (2) zumindest teilweise überlappen, um native Herzklappensegel und/oder infektiöse und/oder thrombotische Auflagerungen/Vegetationen der erkrankten Herzklappe zwischen dem Stentgerüst und den Kompartimentierungsclips (3) zu halten, insbesondere einzuklemmen,
wobei der Katheter eine Katheterspitze aufweist, welche über eine Handhabe des Katheters (20) derart manipulierbar ist, dass das Implantat (1) schrittweise von der Katheterspitze freisetzbar ist, und
wobei die Katheterspitze derart geteilt ausgebildet ist, dass die mindestens zwei Kompartimentierungsclips (3) schrittweise, nacheinander von der Katheterspitze freisetzbar sind, und dann folgend das Stentgerüst freigesetzt werden kann.

9. Katheter nach Anspruch 8,
wobei die geteilte Katheterspitze eine angeschrägte Hülse aufweist, welche dazu ausgebildet ist, die Verankerung während des Einbringens zu überdecken, und welche mit Hilfe der Handhabe zurückziehbar ist, um die Kompartimentierungsclips schrittweise freizusetzen; und/oder wobei der Katheter dazu ausgebildet ist, die Verankerung über die Handhabe zu rotieren, um Kompartimentierungsclips auf die Taschen/Segel/Leaflets der nativen Klappe auszurichten.

10. System zur Behandlung, Prävention und/oder zum Ersatz einer Herzklappe, insbesondere einer entzündeten, infizierten, thrombosierten oder degenerierten Herzklappe, wobei das System eine Verankerung nach einem der Ansprüche 1 bis 7 und einen Katheter nach Anspruch 8 oder 9 aufweist, wobei die Verankerung dazu ausgebildet ist, insbesondere in der Zuführungsform der Verankerung in der Katheterspitze aufnehmbar zu sein, und wobei die Katheterspitze ausgebildet ist, die Verankerung (1), insbesondere in der Zuführungsform der Verankerung, aufzunehmen.

11. Verankerung für Herzklappenprothesen, insbesondere für Herzklappenprothesen zur Behandlung, Prävention, und/oder zum Ersatz einer Herzklappe, insbesondere einer entzündeten, infizierten, thrombosierten oder degenerierten Herzklappe, wobei die Verankerung eine Grundform aufweist, und wobei zum minimal-invasiven Einführen der Verankerung in den Körper des Patienten die Verankerung reversibel und insbesondere durch elastische Umformung und/oder Kompression zumindest von Bereichen oder Komponenten der Verankerung in eine Zuführungsform überführbar ist, wobei die Verankerung folgendes aufweist:
• ein Stentgerüst (2) welches dazu ausgebildet ist, mindestens zwei Herzklappensegel an einer Innenseite zu befestigen, wobei das Stentgerüst ein proximales Ende und ein distales Ende aufweist;
• mindestens zwei Kompartimentierungsclips (3), welche mit dem Stentgerüst verbunden sind und, in der Grundform der Verankerung, das Stentgerüst (2) zumindest teilweise überlappen, um native Segel und/oder infektiöse und/oder thrombotische Auflagerungen/Vegetationen der erkrankten Herzklappe, zwischen dem Stentgerüst (2) und den Kompartimentierungsclips (3) zu halten, insbesondere einzuklemmen wobei die mindestens zwei Kompartimentierungsclips (3) an einen ersten Endbereich, der mit dem Stentgerüst (2) verbunden ist, und einen freien, zweiten Endbereich aufweisen, wobei die Kompartimentierungsclips (3) in der Grundform der Verankerung eine Bogenform zwischen den ersten und zweiten Endbereichen aufweisen, und wobei die Kompartimentierungsclips (3) derart umklappbar sind, dass sich die Kompartimentierungsclips in der Zuführungsform der Verankerung zumindest im Wesentlichen flach zwischen den ersten und zweiten Endbereichen erstrecken;
• eine Trennstruktur, die derart mit dem Stentgerüst (2) und den Kompartimentierungsclips (3) verbunden ist, dass die Trennstruktur, im implantierten Zustand, die zu behandelnden Bereiche der nativen Segel vom Blutstrom trennt.

12. Verankerung nach Anspruch 11,
wobei die Trennstruktur eine erste Strukturoberfläche, welche in der Zuführungsform radial nach innen, insbesondere in Richtung einer Längsachse der Verankerung, gerichtet ist, und eine der ersten Strukturoberfläche gegenüberliegende, zweite Strukturoberfläche aufweist, und wobei zumindest die zweite Strukturoberfläche eine Matrix aufweist, welche antibiotische, antithrombotische und/oder thrombolytische Wirkstoffe enthält und abgeben kann, wobei insbesondere die mindestens eine wirkstoffhaltige Matrix bioresorbierbar ist.

13. Verankerung nach Anspruch 11 oder 12,
wobei das Stentgerüst und die Kompartimentierungsclips aus einem Stück gefertigt sind; oder
wobei das Stentgerüst und/oder die Kompartimentierungsclips aus einem komprimierbaren Werkstoff gefertigt sind; oder
wobei das Stentgerüst und/oder die Kompartimentierungsclips aus einem selbstexpandierbaren Werkstoff gefertigt sind; oder
wobei das Stentgerüst und die Kompartimentierungsclips aus mindestens zwei miteinander verbundenen oder verbindbaren Stücken eines selbstexpandierbaren Werkstoffs gefertigt sind; oder
wobei das Stentgerüst und die Kompartimentierungsclips aus mindestens zwei miteinander verbundenen verbindbaren Stücken gefertigt sind, und das Stentgerüst aus einem ballonexpandierbaren Werkstoff und die Kompartimentierungsclips aus einem selbstexpandierbaren Werkstoff gefertigt sind; oder
wobei das Stentgerüst und/oder die Kompartimentierungsclips aus einer Formgedächtnislegierung, insbesondere Nitinol, bestehen; oder wobei die Kompartimentierungsclips (3) in der Grundform der Verankerung das Stentgerüst (2) zumindest teilweise überlappen, und wobei insbesondere die Verankerung drei Kompartimentierungsclips (3) aufweist.

14. Verankerung nach einem der Ansprüche 1 bis 7 oder 11 bis 13,
wobei die mindestens zwei Kompartimentierungsclips (3) im expandierten Zustand der Verankerung radial voneinander beabstandet und über das Stentgerüst (2) miteinander verbunden sind; und/oder
wobei das Stentgerüst an seinem proximalen Ende eine erste Zellstruktur aus Streben und an seinem distalen Ende eine zweite Zellstruktur aus Streben aufweist, wobei die erste und die zweite Zellstruktur unterschiedlich ausgebildet sind, wobei die erste Zellstruktur insbesondere eine höhere Dichte an Streben aufweist als die zweite Zellstruktur; und/oder
wobei die Verankerung aus einem Rohr aus Formgedächtnislegierung geschnitten ist.

15. Herzklappenprothese, insbesondere für Herzklappenprothese zur Behandlung, Prävention, und/oder zum Ersatz einer Herzklappe, insbesondere einer entzündeten, infizierten, thrombosierten oder degenerierten Herzklappe, wobei die Herzklappenprothese folgendes aufweist:
• eine Verankerung nach einem der Ansprüche 1 bis 7 oder 11 bis 14;
• mindestens zwei Herzklappensegel, welche an der Innenseite des Stentgerüsts der Verankerung befestigt sind.

## Claims

1. An anchoring for heart valve prostheses for the treatment, prevention and/or replacement of a heart valve, in particular an inflamed, infected, thrombosed and/or degenerated heart valve, wherein the anchoring exhibits a primary form and is able to be reversibly transformed into a delivery form for the minimally invasive introduction of the anchoring into the body of the patient and that by elastic deformation and/or compression of at least regions or elements of the anchoring, wherein the anchoring comprises the following:
• a stent framework (2) designed to attach at least two heart valve leaflets to an inner side, wherein the stent framework has a proximal end and a distal end;
• at least two compartmentalization clips (3), each connected to the stent framework at a first end region and having a free second end region;
wherein the compartmentalization clips (3) are arcuately shaped between the first and second end regions in the primary form of the anchoring,
wherein the compartmentalization clips (3) are able to be elastically folded over such that the compartmentalization clips extend in at least substantially flat manner between the first and second end regions in the delivery form of the anchoring,
and wherein the anchoring has a separation structure which is connected to the stent framework (2) and the compartmentalization clips (3) such that the separation structure (5) separates the areas of the native leaflets to be treated from the bloodstream when the anchoring is in the implanted state.

2. The anchoring according to claim 1,
wherein the compartmentalization clips (3) at least partially overlap the stent framework, particularly in the radial and proximal direction of the stent framework, in the primary form of the anchoring.

3. The anchoring according to claim 1 or 2,
wherein the compartmentalization clips comprise arms having an at least partial twisting in the primary form of the anchoring, wherein the compartmentalization clips (2) preferably exhibit a first clip surface and a second clip surface oppositely disposed from the first clip surface, and wherein the first clip surface at the free second end area of the compartmentalization clips is directed at least substantially radially inward, in particular toward a longitudinal axis of the anchoring, in both the primary form as well as the delivery form of the anchoring.

4. The anchoring according to claim 1 or 2,
wherein the compartmentalization clips comprise arms having an at least partial twisting in the primary form of the anchoring, wherein the compartmentalization clips (2) preferably exhibit a first clip surface and a second clip surface oppositely disposed from the first clip surface, and wherein the first clip surface is directed outward and the second clip surface directed inward in the primary form and the first surface is directed inward and the second surface directed outward in the delivery form.

5. The anchoring according to one of claims 1 to 4,
wherein the anchoring is designed to hold, in particular clamp, native heart valve leaflets and/or infectious and/or thrombotic deposits/vegetation of the diseased heart valve between the stent framework and the compartmentalization clips (3) when the anchoring is in an implanted state; and/or
wherein the separation structure exhibits a first structural surface directed radially inward in the delivery form of the anchoring, in particular toward a longitudinal axis of the anchoring, and a second structural surface opposite from the first structural surface, and wherein at least the second structural surface comprises a matrix which contains and can release antibiotic, antithrombotic and/or thrombolytic agents.

6. The anchoring according to one of claims 1 to 5,
wherein the stent framework and the compartmentalization clips are of a single-piece construction; or
wherein the stent framework and/or the compartmentalization clips is/are made from a compressible material; or
wherein the stent framework and/or the compartmentalization clips is/are made from a self-expanding material; or
wherein the stent framework and the compartmentalization clips are made from at least two connected or connectable pieces of a self-expandable material; or
wherein the stent framework and the compartmentalization clips are made from at least two connected or connectable pieces, and the stent framework is made from a balloon-expandable material and the compartmentalization clips from a self-expandable material; or
wherein the stent framework and/or the compartmentalization clips consist of a shape memory alloy, in particular nitinol.

7. The anchoring according to one of claims 1 to 6,
wherein the at least two compartmentalization clips (3) overlap the stent framework (2) in the primary form of the anchoring such that no part of the compartmentalization clips protrudes beyond the distal end of the stent framework (2).

8. A catheter for introducing an anchoring for heart valve prostheses into the body of a patient, wherein the catheter aids in the anchoring being able to be introduced minimally invasively into the body of the patient in a delivery form and transformed into an expanded state in the implantation site on the diseased heart valve, and wherein the anchoring comprises the following:
• a stent framework (2) designed to attach at least two heart valve leaflets to an inner side, wherein the stent framework has a proximal end and a distal end;
• at least two compartmentalization clips (3) connected to the stent framework and which at least partially overlap the stent framework (2) in the primary form of the anchoring in order to hold, in particular clamp, native heart valve leaflets and/or infectious and/or thrombotic deposits/vegetation of the diseased heart valve between the stent framework and the compartmentalization clips (3),
wherein the catheter has a catheter tip able to be manipulated via a handle of the catheter (20) such that the implant (1) is incrementally releasable from the catheter tip, and
wherein the catheter tip is of a divided design such that the at least two compartmentalization clips (2) can be incrementally released from the catheter tip one after the other and the stent framework then released subsequently.

9. The catheter according to claim 8,
wherein the partitioned catheter tip has a tapered sleeve which is designed to cover the anchoring during introduction and which is retractable by means of the handle in order to incrementally release the compartmentalization clips; and/or
wherein the catheter is designed to rotate the anchoring by the handle in order to align the compartmentalization clips with the pockets/leaflets of the native valve.

10. A system for the treatment, prevention and/or replacement of a heart valve, in particular an inflamed, infected, thrombosed or degenerated heart valve, wherein the system comprises an anchoring according to one of claims 1 to 7 and a catheter according to claim 8 or 9, wherein the anchoring is designed to be receivable in the catheter tip, particularly in the delivery form of the anchoring, and wherein the catheter tip is designed to accommodate the anchoring (1), particularly in the delivery form of the anchoring.

11. An anchoring for heart valve prostheses, in particular for heart valve prostheses for the treatment, prevention and/or replacement of a heart valve, in particular an inflamed, infected, thrombosed or degenerated heart valve, wherein the anchoring exhibits a primary form, and wherein the anchoring can be reversibly transformed, and in particular by elastic deformation and/or compression of at least regions or elements of the anchoring, into a delivery form for the minimally invasive introduction of the anchoring into the body of the patient, wherein the anchoring comprises the following:
• stent framework (2) designed to attach at least two heart valve leaflets to an inner side, wherein the stent framework has a proximal end and a distal end;
• at least two compartmentalization clips (3) connected to the stent framework and which at least partially overlap the stent framework (2) in the primary form of the anchoring in order to hold, in particular clamp, native leaflets and/or infectious and/or thrombotic deposits/vegetation of the diseased heart valve between the stent framework (2) and the compartmentalization clips (3), wherein the at least two compartmentalization clips (3) have a first end region which is connected to the stent framework (2) and a free second end region, wherein the compartmentalization clips (3) are able to be folded over such that the compartmentalization clips extend in at least substantially flat manner between the first and second end regions in the delivery form of the anchoring;
• a separation structure connected to the stent framework (2) and the compartmentalization clips (3) such that when in the implanted state, the separation structure separates the areas of the native leaflets to be treated from the bloodstream.

12. The anchoring according to claim 11,
wherein the separation structure exhibits a first structural surface directed radially inward in the delivery form, in particular toward a longitudinal axis of the anchoring, and a second structural surface opposite from the first structural surface, and wherein at least the second structural surface comprises a matrix which contains and can release antibiotic, antithrombotic and/or thrombolytic agents, wherein the at least one active agent matrix is in particular bioresorbable.

13. The anchoring according to claim 11 or 12,
wherein the stent framework and the compartmentalization clips are of a single-piece construction; or
wherein the stent framework and/or the compartmentalization clips is/are made from a compressible material; or
wherein the stent framework and/or the compartmentalization clips is/are made from a self- expanding material; or
wherein the stent framework and the compartmentalization clips are made from at least two connected or connectable pieces of a self-expandable material; or
wherein the stent framework and the compartmentalization clips are made from at least two connected or connectable pieces, and the stent framework is made from a balloon-expandable material and the compartmentalization clips from a self- expandable material; or
wherein the stent framework and/or the compartmentalization clips consist of a shape memory alloy, in particular nitinol; or
wherein the compartmentalization clips (3) at least partially overlap the stent framework (2) in the primary form of the anchoring, and wherein the anchoring in particular comprises three compartmentalization clips.

14. The anchoring according to one of claims 1 to 7 or 11 to 13,
wherein the at least two compartmentalization clips (3) are radially spaced apart from each other and connected together via the stent framework (2) in the expanded state of the anchoring; and/or
wherein the stent framework exhibits a first cell structure made of struts at its proximal end and a second cell structure made of struts at its distal end,
wherein the first and the second cell structure are of different designs,
wherein the first cell structure in particular has a higher density of struts than the second cell structure; and/or
wherein the anchoring is cut from a shape memory alloy tube.

15. A heart valve prosthesis, in particular for heart valve prostheses for the treatment, prevention and/or replacement of a heart valve, particularly an inflamed, infected, thrombosed or degenerated heart valve, wherein the heart valve prosthesis comprises the following:
• an anchoring according to one of claims 1 to 7 or 11 to 14;
• at least two heart valve leaflets affixed to the inner side of the stent framework of the anchoring.

## Revendications

1. Ancrage pour prothèses de valvules cardiaques pour le traitement, la prévention, et/ou le remplacement d'une valvule cardiaque, en particulier d'une valvule cardiaque enflammée, infectée, thrombosée et/ou dégénérée, dans lequel
l'ancrage présente une forme de base et, en vue d'une insertion peu invasive de l'ancrage dans le corps du patient, peut être mis en une forme d'insertion de façon réversible et par déformation et/ou compression élastique d'au moins de zones ou composants de l'ancrage, l'ancrage comprenant ce qui suit :
• une armature d'endoprothèse (2) conçue pour fixer au moins deux feuillets valvulaires sur une face intérieure, l'armature d'endoprothèse ayant une extrémité proximale et une extrémité distale ;
• au moins deux clips de compartimentage (3) qui sont chacun reliés à l'armature d'endoprothèse (2) au niveau d'une première zone d'extrémité et qui présentent une deuxième zone d'extrémité libre,
dans la forme de base de l'ancrage, les clips de compartimentage (3) présentent une forme en arc entre les première et deuxième zones d'extrémité,
les clips de compartimentage (3) peuvent être rabattus élastiquement de telle sorte que, dans la forme d'insertion de l'ancrage, les clips de compartimentage s'étendent au moins sensiblement à plat entre les première et deuxième zones d'extrémité,
l'ancrage présente une structure de séparation qui est reliée à l'armature d'endoprothèse (2) et aux clips de compartimentage (3) de telle sorte qu'à l'état implanté de l'ancrage, la structure de séparation (5) sépare du flux sanguin les zones à traiter des feuillets valvulaires natifs.

2. Ancrage selon la revendication 1,
dans lequel, dans la forme de base de l'ancrage, les clips de compartimentage (3) chevauchent au moins partiellement l'armature d'endoprothèse, en particulier dans les directions radiale et proximale de l'armature d'endoprothèse.

3. Ancrage selon la revendication 1 ou 2,
dans lequel les clips de compartimentage présentent des bras qui, dans la forme de base de l'ancrage, sont torsadés au moins localement,
de préférence, les clips de compartimentage (2) présentent une première surface de clip et une deuxième surface de clip opposée à la première surface de clip, et
aussi bien dans la forme de base que dans la forme d'insertion de l'ancrage, la première surface de clip, au niveau de la deuxième zone d'extrémité libre des clips de compartimentage, est dirigée au moins sensiblement radialement vers l'intérieur, en particulier en direction d'un axe longitudinal de l'ancrage.

4. Ancrage selon la revendication 1 ou 2,
dans lequel les clips de compartimentage présentent des bras qui, dans la forme de base de l'ancrage, sont torsadés au moins localement,
de préférence, les clips de compartimentage (2) présentent une première surface de clip et une deuxième surface de clip opposée à la première surface de clip, et
dans la forme de base, la première surface de clip est dirigée vers l'extérieur et la deuxième surface de clip est dirigée vers l'intérieur, et
dans la forme d'insertion, la première surface est dirigée vers l'intérieur et la deuxième surface est dirigée vers l'extérieur.

5. Ancrage selon l'une des revendications 1 à 4,
dans lequel, dans un état implanté de l'ancrage, l'ancrage est conçu pour maintenir, en particulier pincer, des feuillets valvulaires natifs et/ou des dépôts/végétations infectieux et/ou thrombotiques de la valvule cardiaque malade entre l'armature d'endoprothèse et les clips de compartimentage ; et/ou
la structure de séparation présente une première surface structurelle qui, dans la forme d'insertion de l'ancrage, est dirigée radialement vers l'intérieur, en particulier en direction d'un axe longitudinal de l'ancrage, et une deuxième surface structurelle opposée à la première surface structurelle, et
au moins la deuxième surface structurelle présente une matrice qui contient et peut libérer des principes actifs antibiotiques, antithrombotiques et/ou thrombolytiques.

6. Ancrage selon l'une des revendications 1 à 5,
dans lequel l'armature d'endoprothèse et les clips de compartimentage sont fabriqués d'une seule pièce ; ou
l'armature d'endoprothèse et/ou les clips de compartimentage sont fabriqués en un matériau compressible ; ou
l'armature d'endoprothèse et/ou les clips de compartimentage sont fabriqués en un matériau auto-expansible ; ou
l'armature d'endoprothèse et les clips de compartimentage sont fabriqués à partir d'au moins deux pièces d'un matériau auto-expansible reliées ou pouvant être reliées entre elles ; ou
l'armature d'endoprothèse et les clips de compartimentage sont fabriqués à partir d'au moins deux pièces reliées ou pouvant être reliées entre elles, et l'armature d'endoprothèse est fabriquée à partir d'un matériau expansible par ballonnet et les clips de compartimentage sont fabriqués à partir d'un matériau auto-expansible ; ou
l'armature d'endoprothèse et/ou les clips de compartimentage sont constitués d'un alliage à mémoire de forme, en particulier de nitinol.

7. Ancrage selon l'une des revendications 1 à 6,
dans lequel, dans la forme de base de l'ancrage, lesdits au moins deux clips de compartimentage (3) chevauchent l'armature d'endoprothèse (2) de telle sorte qu'aucune partie des clips de compartimentage ne dépasse de l'extrémité distale de l'armature d'endoprothèse (2).

8. Cathéter pour l'insertion d'un ancrage pour prothèses de valvules cardiaques dans le corps d'un patient, le cathéter permettant d'insérer l'ancrage, dans une forme d'insertion, de manière peu invasive dans le corps du patient et de le mettre dans un état explanté sur le site d'implantation sur la valvule cardiaque malade, et l'ancrage comprenant ce qui suit :
• une armature d'endoprothèse (2) qui est conçue pour fixer au moins deux feuillets valvulaires ou leaflets sur une face intérieure, l'armature d'endoprothèse présentant une extrémité proximale et une extrémité distale ;
• au moins deux clips de compartimentage (3) qui sont reliés à l'armature d'endoprothèse et qui, dans une forme de base de l'ancrage, chevauchent au moins partiellement l'armature d'endoprothèse (2) afin de maintenir, en particulier pincer, des feuillets valvulaires natifs et/ou des dépôts/végétations infectieux et/ou thrombotiques de la valvule cardiaque malade entre l'armature d'endoprothèse et les clips de compartimentage (3) ;
dans lequel le cathéter présente une pointe de cathéter qui peut être manipulée par une manette du cathéter (20) de telle sorte que l'implant (1) peut être libéré progressivement de la pointe du cathéter, et
la pointe du cathéter est réalisée de façon divisée de manière à permettre de libérer progressivement, l'un après l'autre, lesdits au moins deux clips de compartimentage (3) de la pointe du cathéter, et de libérer ensuite l'armature d'endoprothèse.

9. Cathéter selon la revendication 8,
dans lequel la pointe divisée du cathéter comprend un manchon biseauté qui est conçu pour recouvrir l'ancrage pendant l'insertion et qui peut être retiré à l'aide de la manette afin de libérer progressivement les clips de compartimentage ; et/ou
le cathéter est conçu pour faire tourner l'ancrage par l'intermédiaire de la manette afin d'aligner les clips de compartimentage avec les valvules/feuillets/leaflets de la valve native.

10. Système de traitement, de prévention et/ou de remplacement d'une valvule cardiaque, en particulier d'une valvule cardiaque enflammée, infectée, thrombosée ou dégénérée, ledit système comprenant un ancrage selon l'une des revendications 1 à 7 et un cathéter selon la revendication 8 ou 9,
dans lequel l'ancrage est conçu pour être reçu dans la pointe du cathéter, en particulier dans la forme d'insertion de l'ancrage, et
la pointe du cathéter est conçue pour recevoir l'ancrage (1), en particulier dans la forme d'insertion de l'ancrage.

11. Ancrage pour prothèses de valvules cardiaques, en particulier pour prothèses de valvules cardiaques, pour le traitement, la prévention et/ou le remplacement d'une valvule cardiaque, en particulier d'une valvule cardiaque enflammée, infectée, thrombosée ou dégénérée, dans lequel l'ancrage présente une forme de base et, en vue de l'insertion peu invasive de l'ancrage dans le corps du patient, l'ancrage peut être mis en une forme d'insertion de façon réversible et en particulier par déformation et/ou compression élastique d'au moins de zones ou composants de l'ancrage, l'ancrage comprenant ce qui suit :
• une armature d'endoprothèse (2) conçue pour fixer au moins deux feuillets valvulaires sur une face intérieure, l'armature d'endoprothèse ayant une extrémité proximale et une extrémité distale ;
• au moins deux clips de compartimentage (3) qui sont reliés à l'armature d'endoprothèse et qui, dans la forme de base de l'ancrage, chevauchent au moins partiellement l'armature d'endoprothèse (2) afin de maintenir, en particulier pincer, des feuillets natifs et/ou des dépôts/végétations infectieux et/ou thrombotiques de la valve cardiaque malade entre l'armature d'endoprothèse (2) et les clips de compartimentage (3),
lesdits au moins deux clips de compartimentage (3) présentent une première zone d'extrémité reliée à l'armature d'endoprothèse (2) et une deuxième zone d'extrémité libre,
dans la forme de base de l'ancrage, les clips de compartimentage (3) présentent une forme en arc entre les première et deuxième zones d'extrémité,
les clips de compartimentage (3) peuvent être rabattus de telle sorte que, dans la forme d'insertion de l'ancrage, les clips de compartimentage s'étendent au moins sensiblement à plat entre les première et deuxième zones d'extrémité,
• une structure de séparation qui est reliée à l'armature d'endoprothèse (2) et aux clips de compartimentage (3) de telle sorte que la structure de séparation, à l'état implanté, sépare du flux sanguin les zones à traiter des feuillets natifs.

12. Ancrage selon la revendication 11,
dans lequel la structure de séparation présente une première surface structurelle qui, dans la forme d'insertion, est dirigée radialement vers l'intérieur, en particulier en direction d'un axe longitudinal de l'ancrage, et une deuxième surface structurelle opposée à la première surface structurelle, et
au moins la deuxième surface structurelle présente une matrice qui contient et peut libérer des principes actifs antibiotiques, antithrombotiques et/ou thrombolytiques,
en particulier, ladite au moins une matrice contenant des principes actifs est biorésorbable.

13. Ancrage selon la revendication 11 ou 12,
dans lequel l'armature d'endoprothèse et les clips de compartimentage sont fabriqués d'une seule pièce ; ou
l'armature d'endoprothèse et/ou les clips de compartimentage sont fabriqués en un matériau compressible ; ou
l'armature d'endoprothèse et/ou les clips de compartimentage sont fabriqués en un matériau auto-expansible ; ou
l'armature d'endoprothèse et les clips de compartimentage sont fabriqués à partir d'au moins deux pièces d'un matériau auto-expansible reliées ou pouvant être reliées entre elles ; ou
l'armature d'endoprothèse et les clips de compartimentage sont fabriqués à partir d'au moins deux pièces reliées ou pouvant être reliées entre elles, et l'armature d'endoprothèse est fabriquée à partir d'un matériau expansible par ballonnet et les clips de compartimentage sont fabriqués à partir d'un matériau auto-expansible ; ou
l'armature d'endoprothèse et/ou les clips de compartimentage sont constitués d'un alliage à mémoire de forme, en particulier de nitinol ; ou dans la forme de base de l'ancrage, les clips de compartimentage (3) chevauchent au moins partiellement l'armature d'endoprothèse, et en particulier, l'ancrage comprend trois clips de compartimentage (3).

14. Ancrage selon l'une des revendications 1 à 7 ou 11 à 13,
dans lequel lesdits au moins deux clips de compartimentage (3) sont espacés radialement l'un de l'autre à l'état expansé de l'ancrage et sont reliés l'un à l'autre par l'intermédiaire de l'armature d'endoprothèse (2) ; et/ou l'armature d'endoprothèse présente à son extrémité proximale une première structure cellulaire constituée d'entretoises et à son extrémité distale une deuxième structure cellulaire constituée d'entretoises, les première et deuxième structures cellulaires étant formées différemment, en particulier, la première structure cellulaire présentant une densité d'entretoises supérieure à celle de la deuxième structure cellulaire ; et/ou
l'ancrage est découpé dans un tube en alliage à mémoire de forme.

15. Prothèse de valvule cardiaque, en particulier prothèse de valvule cardiaque pour le traitement, la prévention, et/ou le remplacement d'une valve cardiaque, en particulier d'une valve cardiaque enflammée, infectée, thrombosée ou dégénérée, ladite prothèse de valvule cardiaque comprenant ce qui suit :
• un ancrage selon l'une des revendications 1 à 7 ou 11 à 14 ;
• au moins deux feuillets valvulaires fixés sur la face intérieure de l'armature d'endoprothèse de l'ancrage.
